# EUROPEAN PATENT SPECIFICATION

(11) **EP 3 153 291 B1**
(45) Date of publication and mention of the grant of the patent: **25.07.2018**
(21) Application number: 16192486.5
(22) Date of filing: 05.10.2016
(51) Int. Cl.: B29C 33/38, B29C 33/40, B29C 33/42, B29C 33/68

(54) **METHOD OF MANUFACTURING MOLD AND METHOD OF MANUFACTURING PATTERN SHEET**
VERFAHREN ZUR HERSTELLUNG EINER FORM UND VERFAHREN ZUR HERSTELLUNG EINER MUSTERSCHICHT
PROCÉDÉ DE FABRICATION DE MOULE ET PROCÉDÉ DE FABRICATION DE FEUILLE DE MOTIF

(30) Priority: 06.10.2015 JP 2015198257
(43) Date of publication of application: 12.04.2017
(73) Proprietor: FUJIFILM Corporation, Tokyo 106-8620 (JP)
(72) Inventor: OGAWA, Shotaro, Kanagawa, 258-8577 (JP); USA, Toshihiro, Kanagawa, 258-8577 (JP); FUJITA, Atsushi, Kanagawa, 258-8577 (JP); MOCHIZUKI, Aya, Kanagawa, 258-8577 (JP)
(74) Representative: Klunker IP Patentanwälte PartG mbB

(56) References cited:
- EP-A1- 2 921 201
- WO-A1-2014/196522
- JP-A- 2010 069 252
- JP-A- 2011 224 332
- JP-A- 2015 136 528
- US-A1- 2009 194 908

## Description

### BACKGROUND OF THE INVENTION

### Field of the Invention

The present invention relates to a method of manufacturing a mold and a method of manufacturing a pattern sheet, and more particularly to a method of manufacturing a mold and a method of manufacturing a pattern sheet, capable of reliably manufacturing a mold and a pattern sheet each with a uniform shape.

### Description of the Related Art

Conventionally, most of the methods of applying medicine through a surface of a living body, such as skin and mucosa, are methods of adhesion of a liquid substance or a powdery substance. Unfortunately, since an adhesion area of these substances is limited to a surface of skin, an attached medicine and the like may be removed due to sweating or contact with a foreign material, whereby it is difficult to apply a proper amount of medicine. In such a method using permeation caused by diffusion of medicine, it is difficult to reliably control permeation depth to allow medicine to deeply permeate skin, whereby it is difficult to acquire a sufficient medical effect.

For this reason, a transdermal absorption sheet having a needle-like protruding section with a high aspect ratio (also referred to as fine needles or micro needles) containing medicine, is used for a method of injecting the medicine into skin by inserting the fine needles into the skin.

As a method of manufacturing a functional film having this kind of structure with a high aspect ratio, Japanese Patent Application Laid-Open No. 2015-100401, for example, describes a method of forming a needle-like pad having a thin film in which: a thin film made of TiN is provided on a surface of a protrusion; a plate with recessed portions made of silicone resin is formed from the needle-like pad having a thin film; and a needle-like pad is formed by using the plate with recessed portions. EP 2 921 201 A1 discloses a method of manufacturing a mold having a recessed pattern comprising the steps of applying a thermosetting resin solution on a surface of a model, providing a gap adjustment mechanism around the model, pressing a base plate against the gap adjustment mechanism, heating and curing the thermosetting resin film to form a mold, releasing the base plate and then releasing the mold.
WO 2014/196522 A1 discloses a method of manufacturing a molded product, wherein a release sheet is brought into contact with thermosetting resin in order to improve releaseability of a mold half.

### SUMMARY OF THE INVENTION

The needle-like protruding section of a transdermal sheet is inserted into skin, and thus is required to have a pungent tip shape. Unfortunately, there is a problem that sharping a tip shape of the needle-like protruding section may cause the needle-like protruding section of a model (male mold) or a molding, particularly a needle tip, to be easily deformed during manufacturing. Since a finer and more complex pattern shape increases costs for a model and a master plate for manufacturing a mold, and their durability is limited, it is preferable to manufacture a large number of molds. If the master plate is deformed, a mold and a pattern sheet that is a molding from the mold cannot be manufactured to result in increasing costs as a whole. In addition, deformation of a molding deteriorates productivity of the molding.

The present invention is made in light of the above-mentioned circumstances, and has an object to provide a method of manufacturing a mold and a method of manufacturing a pattern sheet which can achieve low costs and high productivity without damaging a needle tip of a protrusion pattern and a molding with a protrusion pattern.

To achieve the object above, the present invention provides a method of manufacturing a mold having a recessed pattern, the method including the steps of claim 1.

According to the present invention, the gap adjustment mechanism is provided to adjust a thickness of the mold, and the gap adjustment mechanism has a height at which the tip of the protrusion pattern sticks into the separation sheet when the base plate with which the separation sheet is in close contact is pressed. This configuration enables a through-hole to be provided in the recessed pattern of the mold, and enables a liquid to be easily injected into the recessed pattern when a reversed shape of the recessed pattern is manufactured by using the mold.

The tip of the protrusion pattern of the model is stuck into the separation sheet, but is not stuck into the base plate. As a result, the tip of the protrusion pattern is not damaged or deformed when the base plate is released, and thus the model can be used for a long time to result in reducing manufacturing costs.

A plurality of the molds is joined to form an assembled mold. According to this aspect, since a pattern sheet is formed after a plurality of molds are joined to form an assembled mold, a pattern sheet with a large area of protrusion patterns can be manufactured in one production of a pattern sheet, whereby the productivity can be improved.

According to the method of manufacturing a mold and the method of manufacturing a pattern sheet of the present invention, a mold and a pattern sheet can be manufactured without deforming a shape of a tip of a protrusion pattern of a model and the pattern sheet. Thus, the pattern sheet can be uniformly manufactured at low cost and with high productivity.

In another aspect of the present invention, it is preferable that the separation sheet is a fluorocarbon rubber sheet, or a silicone resin sheet to which a release treatment has been applied.

This aspect limits a material of the separation sheet, and enables the base plate to be easily released from the mold without damaging the tip of the protrusion pattern of the model, by using the materials described above for the separation sheet.

In yet another aspect of the present invention, it is preferable that the base plate is provided with an air suctioning mechanism and an air injecting mechanism to bring the separation sheet into close contact with the base plate or release the separation sheet from the base plate.

According to this aspect, since the base plate is provided with the air suctioning mechanism and the air injecting mechanism, the base plate can be easily released from the separation sheet. Thus, the tip of the protrusion pattern of the model can be prevented from being damaged when the base plate is released.

In yet another aspect of the present invention, it is preferable that the model having the protrusion pattern is a metal electroformed model formed by electroforming from a first mold having a recessed pattern which has a reversed shape of the protrusion pattern.

According to this aspect, since an electroformed model formed by electroforming from the first mold serves as the model having a protrusion pattern, a plurality of electroformed models can be formed from the first mold. Thus, since manufacturing costs of the model having a protrusion pattern can be reduced, manufacturing costs can be reduced as a whole.

In yet another aspect of the present invention, it is preferable that the model having the protrusion pattern includes a plurality of areas each provided with the protrusion pattern, and that the thermosetting resin solution is applied by a slit nozzle in a width of the protrusion pattern.

According to this aspect, since the thermosetting resin film is applied by using a slit nozzle in a width of the protrusion pattern, an extra application of the thermosetting resin solution can be prevented.

To achieve the object above, the present invention provides a method of manufacturing a pattern sheet having a protrusion pattern, the method including the steps of: supplying a polymer solution to a recessed pattern of a mold manufactured by the method of manufacturing a mold described above; drying the polymer solution to form a polymer sheet; and releasing the polymer sheet from the mold.

According to the present invention, since a pattern sheet is manufactured by using the mold described above, a pattern sheet, which is a duplicate of the model with a uniform shape, can be manufactured.

In yet another aspect of the present invention, it is preferable that the polymer solution is a water-soluble material.

According to this aspect, since the polymer solution forming the pattern sheet is a water-soluble material, medicine can be easily injected when the protrusion pattern formed on the pattern sheet is inserted into skin.

### BRIEF DESCRIPTION OF THE DRAWINGS

Fig. 1 is a process chart illustrating a procedure of manufacturing a model having a protrusion pattern;
Fig. 2A to 2C are diagrams illustrating processes in a procedure of forming a silicone resin film;
Figs. 3A to 3F are diagrams illustrating processes in a procedure of manufacturing a mold;
Fig. 4 is a plan view of the model after disposing spacers;
Fig. 5 illustrates positions of the protrusion pattern of the model and a separation sheet during manufacturing of a mold;
Figs. 6A to 6F are diagrams illustrating processes in a procedure of manufacturing a pattern sheet; and
Fig. 7 is a schematic side view of an assembled mold, obtained with the method of the current invention.

### DETAILED DESCRIPTION OF THE PREFERRED EMBODIMENTS

Hereinafter, a method of manufacturing a mold and a method of manufacturing a pattern sheet in accordance with the present invention will be described with reference to accompanying drawings. In the present specification, the term, "to" between numeric values is used so that the numeric values described at the front and back of the term, "to" indicate a lower limit value and an upper limit value, respectively. In addition, an example using silicone resin as an example of thermosetting resin will be described below.

The present invention is a technique of manufacturing a mold (female mold) from a model (male mold) having a protrusion pattern with no defect, the mold having a reversed shape of the protrusion pattern, without damaging a needle tip of the protrusion pattern, and a technique of manufacturing a pattern sheet by using the mold, the pattern sheet being a duplicate of the model having the protrusion pattern. Specifically, a silicone resin solution for forming a mold is applied on a surface of a model (male mold) 40 having a protrusion pattern 42 to form a silicone resin film 60. Next, a separation sheet 66 is brought into close contact with one face of the base plate 64, and a separation sheet 66 side of the base plate 64 is pressed on the silicone resin film 60 and a gap adjustment mechanism (spacer 52). In this state, the silicone resin film 60 is heated and cured to form a mold (female mold) 68. Then, after the base plate 64 is released, the mold 68 is released from the model 40 while the separation sheet 66 is attached to the mold 68. Last, the separation sheet 66 is released from the mold 68 to manufacture the mold 68. In addition, a pattern sheet 76 is manufactured by using the mold 68. Each of steps will be described below.

### (Method of Manufacturing Mold)

### [Step of manufacturing First Mold]

A first mold 20 includes a recessed pattern 22 having a reversed shape of the model 40 and is used to manufacture the model 40 having the protrusion pattern 42 that has a reversed shape of the mold 68. The first mold 20 is manufactured from a master plate 10 provided with a protrusion pattern 12, by using a resin.

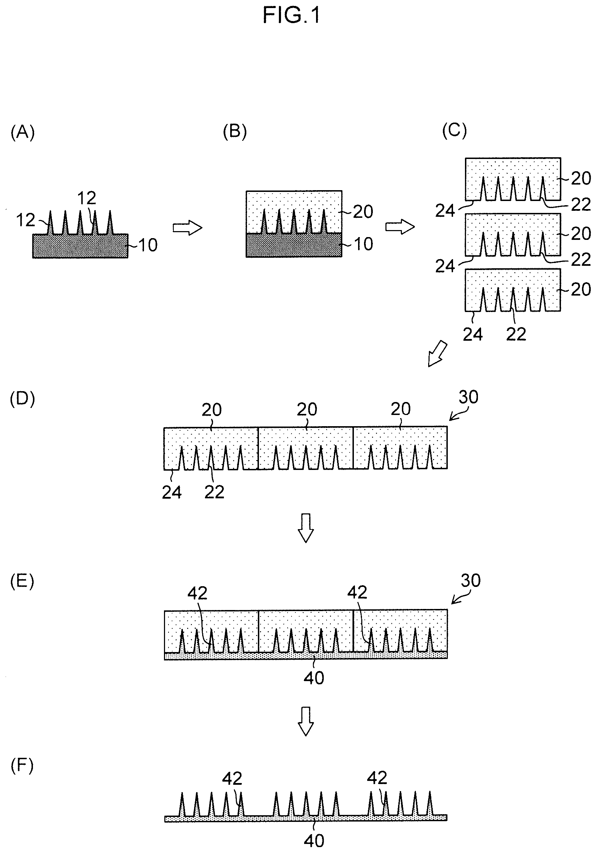

First, as illustrated in Part (A) in Fig. 1, the master plate 10 provided with the protrusion pattern 12 is prepared. A method of manufacturing the master plate 10 provided with the protrusion pattern 12 is not particularly limited, but, for example, the master plate 10 can be manufactured as follows. A plurality of protrusion patterns 12 can be manufactured in a surface of the master plate 10 by cutting a metal base plate, such as Ni, by machine cutting with a cutting tool such as a diamond tool.

In another method, after a photoresist is applied on a Si base plate, the photoresist is exposed and developed. Then, etching such as reactive ion etching (RIE) is applied to a surface of the master plate 10 to form the protrusion pattern 12 in the surface thereof. When etching such as RIE is applied to form the protrusion pattern 12 on the surface of the master plate 10, the protrusion pattern can be formed by applying the etching to the Si base plate in an oblique direction while the Si base plate is rotated.

Next, as illustrated in Part (B) and Part (C) in Fig. 1, a plurality of first molds 20 each having the recessed pattern 22 are manufactured by using the master plate 10. The first mold 20 can be manufactured by the following methods using a resin. In a first method, a silicone resin in which a hardening agent is added into polydimethylsiloxane (PDMS), such as Sylgard 184 of Dow Corning Corp. (Sylgard: registered trademark), is poured to the master plate 10, and is heated at 100°C and cured, thereby forming the first mold 20 having the recessed pattern 22 from the master plate 10, the first mold 20 having a reversed shape of the protrusion pattern.

In a second method, an ultraviolet light curable resin, which is curable by being irradiated with ultraviolet light, is poured to the master plate 10 and is irradiated with ultraviolet light in a nitrogen atmosphere, and then is the first mold 20 is released from the master plate 10. In a third method, a solution in which a plastic resin, such as polystyrene and polymethyl methacrylate (PMMA), is dissolved in an organic solvent is poured to the master plate 10 being coated with a release agent and is cured by drying so as to volatize the organic solvent, and then the first mold 20 is released from the master plate 10.

It is preferable to use a resin, such as an ultraviolet light curable resin or a thermoplastic resin, as a material forming the first mold 20. Using an ultraviolet light curable resin or a thermoplastic resin enables the first mold 20 to be easily manufactured as well as the protrusion pattern 12 of the master plate 10 to be uniformly formed.

### [Step of manufacturing First Assembled Mold]

Next, as illustrated in Part (D) in Fig. 1, a plurality of first molds 20 manufactured from the master plate 10 are joined to increase an area of a face 24 provided with the recessed pattern 22 of the first mold 20, thereby manufacturing a first assembled mold 30.

The first assembled mold 30 can be manufactured by joining the plurality of first molds 20. The first molds 20 can be joined by using an adhesive, for example. Since the first assembled mold 30 serves as a form of the model 40 having the protrusion pattern 42 to be manufactured in a subsequent step, it is preferable to form the first assembled mold 30 while flatness of the face 24 provided with the recessed pattern 22 is secured. If the flatness of the face 24 provided with the recessed pattern 22 is not secured, a level difference is unfavorably formed in an electroformed model, a mold, and a pattern sheet, which are to be manufactured in subsequent steps.

Although a method of manufacturing the first assembled mold 30 while securing flatness of the recessed pattern 22 is not particularly limited, for example, the flatness of the first assembled mold 30 can be secured by: making the face 24 provided with the recessed pattern 22 of the first mold 20 face down above the base plate, disposing the first mold 20 at a position where the face 24 is brought into contact with the base plate, and adhering the face 24 on the base plate with an adhesive. Since the flatness of the first assembled mold 30 is secured by using the base plate as described above, it is possible to prevent generation of a level difference with an adhesive between the first molds 20 in the first assembled mold 30, a deformation therebetween, and the like, whereby the flatness of the first assembled mold 30 can be secured.

The first assembled mold 30 may be manufactured, but doesn't necessarily have to be manufactured. If the first assembled mold 30 is not manufactured, a model having a protrusion pattern is manufactured from the first mold 20. However, manufacturing the first assembled mold 30 is preferable because a model with a large area can be manufactured by one electroforming. Manufacturing the first assembled mold 30 is also preferable in subsequent steps because a mold can be manufactured by using the model with a large area, and a pattern sheet can be manufactured from the mold to enable productivity of the pattern sheet to be improved.

### [Step of manufacturing Model having Protrusion Pattern]

Part (E) in Fig. 1 illustrates a step of manufacturing the model 40 having the protrusion pattern 42 by using the first assembled mold 30. Hereinafter, a method of manufacturing a model having a protrusion pattern by electroforming will be described, for example.

In the electroforming, first, a conductive treatment is applied to the first assembled mold 30. Metal (e.g. nickel) is spattered on the first molds 20 of the first assembled mold 30, and attached to a surface of each of the first molds 20 of the first assembled mold 30 and the recessed pattern 22.

Next, the first assembled mold having been subjected to the conductive treatment is held at a negative pole. A metal pellet is held in a metal case to serve as a positive pole. The negative pole holding the first assembled mold 30 and the positive pole holding the metal pellet are immersed in electroforming liquid and are energized to embed metal in recessed pattern 22 of the first assembled mold 30 to form the model 40. Then, the model 40 having the protrusion pattern 42 (hereinafter also referred to as an "electroformed model 40") is manufactured by releasing it from the first assembled mold (refer to Part (F) in Fig. 1).

The model 40 having the protrusion pattern 42 is not limited to a model manufactured by the method illustrated in Part (A) to Part (F) in Fig. 1, and a model manufactured by the method of manufacturing the master plate 10 also can be used as the model 40. However, since a finer and more complex pattern shape increases manufacturing cost of the master plate 10, manufacturing a plurality of master plates is costly. In addition, each of the master plates may have a protrusion pattern different in a shape. Thus, as illustrated in Part (A) to Part (F) in Fig. 1, manufacturing the model 40 by manufacturing a plurality of first molds 20 from the master plate 10 enables the model 40 to be manufactured at low cost.

### [Step of forming Silicone Resin Film]

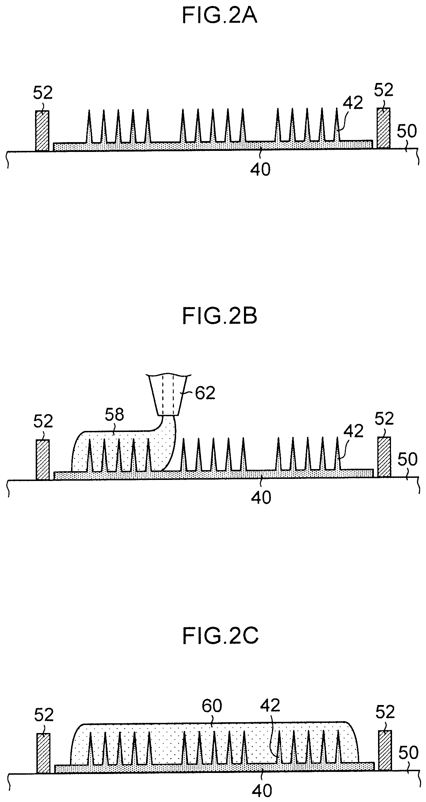

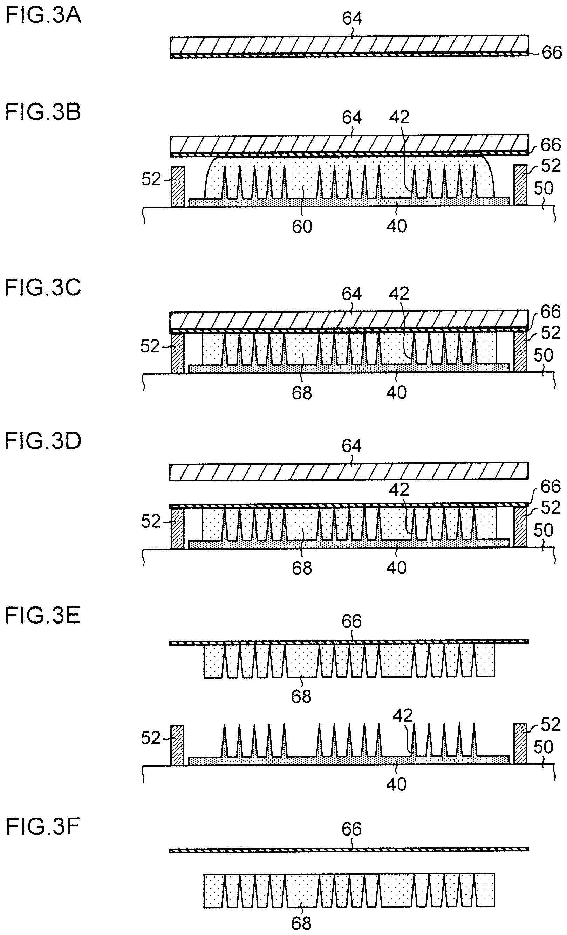

Subsequently, a method of manufacturing a mold by using the electroformed model 40 manufactured as described above will be described. Figs. 2A to 2C illustrate steps of forming a silicone resin film, and Figs. 3A to 3F illustrate steps of molding a mold and releasing the mold from the electroformed model.

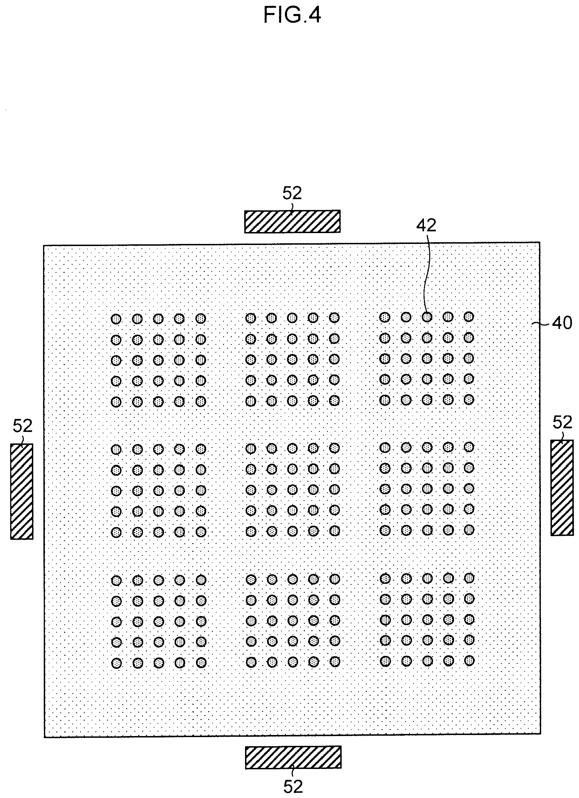

First, as illustrated in Fig. 2A, the electroformed model 40 is disposed on a base 50. In addition, spacers 52 are disposed outside the electroformed model 40 on the base 50, as gap adjustment mechanisms. Adjusting height of each of the spacers 52 enables a distance between the electroformed model 40 and the base plate 64 to be adjusted in a subsequent step of pressing the silicone resin film 60 by the base plate 64, thereby adjusting thickness of the manufactured mold 68. Fig. 4 is a plan view of the model after the spacers are disposed. While the spacer 52, as illustrated in Fig. 4, is provided outside a central portion of each side of the electroformed model 40 of a quadrangular shape, the position of each spacer 52 is not limited to the above as long as the thickness of the mold can be made uniform when the base plate 64 is brought into contact with the spacers 52. The spacers 52 may be provided all around the electroformed model 40, or may be provided outside respective corners of the electroformed model 40.

The spacer 52 can be made of metal, such as stainless used steel (SUS) and aluminum, and glass. The spacer 52 is preferably made of a material with high rigidity because bringing the base plate 64 into contact with spacer 52 enables thickness of the silicone resin film 60 to be adjusted to a height of the spacer 52.

Next, as illustrated in Figs. 2B and 2C, a silicone resin solution 58 is applied on the electroformed model 40 and the silicone resin film 60 is formed.

A method of applying the silicone resin solution 58 is not particularly limited, and an application using a spin coater is available. If there are a plurality of areas in each of which the protrusion pattern 42 is formed, the silicone resin solution 58 can be applied to each area having the protrusion pattern 42 by a slit nozzle 62 in a width of the protrusion pattern 42. Applying the silicone resin solution 58 to each area having the protrusion pattern 42 enables the silicone resin solution 58 to be applied to only a position where a mold is manufactured, whereby an unnecessary application of the silicone resin solution can be prevented.

### [Step of defoaming Silicone Resin Film]

Subsequently, the silicone resin film 60 formed on the electroformed model 40 is reduced in pressure to defoam the silicone resin film 60. Defoaming of the silicone resin film 60 is performed by placing the silicone resin film 60 in a pressure reduction device (not illustrated) together with the base 50, the electroformed model 40, the spacers 52, and the like, and reducing a pressure in the pressure reduction device.

Defoaming of the silicone resin film 60 can be checked visually or by image recognition with a charge-coupled device (CCD) camera. Defoaming the silicone resin film 60 enables air contained in the silicone resin film to be eliminated, and thus a shape of the silicone resin film 60 at the time of molding can be uniform.

### [Step of preparing Base Plate]

Next, as illustrated in Fig. 3A, the separation sheet 66 is brought into close contact with one face of the base plate 64. The face with which the separation sheet 66 is in close contact becomes a face which presses the silicone resin film 60.

The base plate 64 can be made of metal, a porous ceramic, or the like. When these materials are used for the base plate 64, the mold 68 to be formed can have a uniform height.

The separation sheet 66 can be made from a fluorocarbon rubber sheet, a Teflon (registered trademark) sheet, a silicone resin sheet to which a release treatment has been applied, and the like. The release treatment can be performed by an application of a fluorine mold release agent, or the like. It is preferable that thickness of the separation sheet 66 is not less than 0.1 mm and not more than 0.5 mm.

The base plate 64 and the separation sheet 66 can be brought into close contact with each other and released from each other by using an air suctioning mechanism and an air injecting mechanism (not illustrated). The base plate 64 and the separation sheet 66 can be brought into close contact with each other by suctioning air from a face opposite to the separation sheet 66 on the base plate 64 with the air suctioning mechanism, and can be released from each other by supplying air with the air injecting mechanism. A vacuum pump can be used as the air suctioning mechanism, and a blower can be used as the air injecting mechanism. Switching between the air suctioning mechanism and the air injecting mechanism can be performed by using a change-over valve, for example. When the base plate and the separation sheet are brought into close contact with each other by using the air suctioning mechanism and when they are released from each other by the air injecting mechanism, it is preferable that the base plate 64 is made of a porous ceramic. When the base plate 64 is made of a porous material, the base plate 64 and the separation sheet 66 can be brought into close contact with each other and released from each other by suctioning and suppling air from the opposite side to the face on which the separation sheet 66 is attached, of the base plate 64.

### [Step of pressing Base Plate]

Subsequently, as illustrated in Fig. 3B, the base plate 64 is brought into contact with the silicone resin film 60. Then, the base plate 64 is pressed on the spacers 52 as illustrated in Fig. 3C to press also the silicone resin film 60, thereby adjusting the thickness of the silicone resin film 60, that is, the thickness of the mold 68 to be formed.

### [Step of forming Mold]

Next, while the base plate 64 is in contact with the spacer 52 and the silicone resin film 60, the silicone resin film 60 is heated and cured to form the mold 68. The silicone resin film 60 can be heated with an aluminum heater, an infrared ray heater, a ceramic heater, an induction heating (IH) heater, or the like.

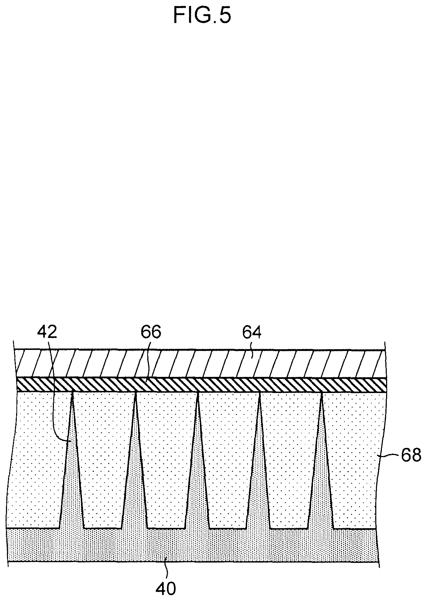

Fig. 5 illustrates positions of the protrusion pattern 42 and the separation sheet 66 during forming of the mold 68. Height of each of the spacers 52 is adjusted so that tips of the protrusion pattern 42 of the electroformed model 40 stick into the separation sheet 66 when the silicone resin film 60 is cured, and then the base plate 64 is pressed.

In the present invention, a through-hole is provided in each of recessed portions of a recessed pattern 70 of the mold 68. Proving the through-hole enables a polymer solution to be easily injected into the recessed portions of the mold when a pattern sheet is manufactured. The protrusion pattern 42 of the electroformed model 40 penetrates the silicone resin film 60 to stick into the separation sheet 66, thereby forming the through-hole in the mold 68.

Positions of the tip of the protrusion pattern 42 and the separation sheet can be adjusted by a height of the spacer 52. If the thickness of the separation sheet 66 is set within a range of not less than 0.1 mm and not more than 0.5 mm, as described above, as well as is set less than the height of the protrusion pattern by about 0.03 mm to 0.1 mm, the tip of the protrusion pattern 42 can be positioned to stick into the separation sheet 66. If the thickness of the separation sheet 66 is less than the range above, the tip of the protrusion pattern 42 is brought into contact with the base plate 64 when the base plate 64 is pressed, whereby the tip of the protrusion pattern 42 of the electroformed model 40 may be damaged or broken. If the mold 68 is manufactured by using the electroformed model 40 with a broken tip of the protrusion pattern 42, the mold 68 does not have a reversed shape of the protrusion pattern 12 of the electroformed model 40. Since the broken electroformed model 40 cannot be used for subsequent manufacturing of the mold 68, the tip of the protrusion pattern 42 of the electroformed model 40 needs to be prevented from being deformed in manufacturing.

In addition, providing the separation sheet 66 between the base plate 64 and the silicone resin film 60 enables the base plate 64 and the silicone resin film 60 to be easily released from each other. Since the base plate 64 is made of the material described above, the base plate 64 is inflexible, and thus if the mold 68 is released from the electroformed model 40 while the base plate 64 is disposed, the tip of the protrusion pattern 42 of the electroformed model 40 may be damaged or broken. Providing the separation sheet 66 enables the mold 68 and the base plate 64 to be easily released from each other, and thus the mold can be manufactured while deformation of the protrusion pattern 42 is prevented.

In manufacturing of a pattern sheet, manufacturing a master plate and the like by cutting is costly. Thus, if a large number of molds can be manufactured from one master plate, a manufacturing cost of a pattern sheet can be reduced. In a case where a pattern sheet is used for medical supplies and the like, it is preferable that the pattern sheet is used only once in consideration of a cleaning cost and the like. Accordingly, a large number of molds need to be manufactured. According to the present embodiment, because the deformation of the electroformed model 40 can be can prevented during manufacturing of the mold 68, the mold 68 can be repeatedly manufactured by using the electroformed model 40, whereby a manufacturing cost of the mold 68 can be reduced, and the productivity can be improved.

### [Step of releasing Base Plate]

Subsequently, as illustrated in Fig. 3D, the base plate 64 is released from the mold 68. The separation sheet 66 is still attached to the mold 68.

As illustrated in Fig. 5, the tip of the protrusion pattern 42 of the electroformed model 40 sticks into only the separation sheet 66 in the present embodiment, and thus the base plate 64 can be released without damaging a shape of the tip of the protrusion pattern 42 of the electroformed model 40 when the base plate 64 is released. If the tip of the protrusion pattern 42 of the electroformed model 40 sticks into the base plate 64, the base plate 64 is released while rubbing on the tip of the protrusion pattern 42 when the base plate 64 is released. As a result, the tip may be unfavorably damaged or broken.

Here, because the base plate 64 includes the air injecting mechanism, only the base plate 64 can be easily released by supplying air with the air injecting mechanism.

### [Step of releasing Mold]

Subsequently, as illustrated in Fig. 3E, the mold 68 to which the separation sheet 66 is attached is released from the electroformed model 40. Since the base plate 64 is released and the tip of the protrusion pattern 42 sticks into the separation sheet, the mold 68 can be released while the tip of the protrusion pattern 42 is protected.

Last, as illustrated in Fig. 3F, the mold 68 having the recessed pattern 70 which has a reversed shape of the protrusion pattern 42 of the electroformed model 40 can be manufactured by releasing the separation sheet 66.

### [Method of Manufacturing Pattern Sheet]

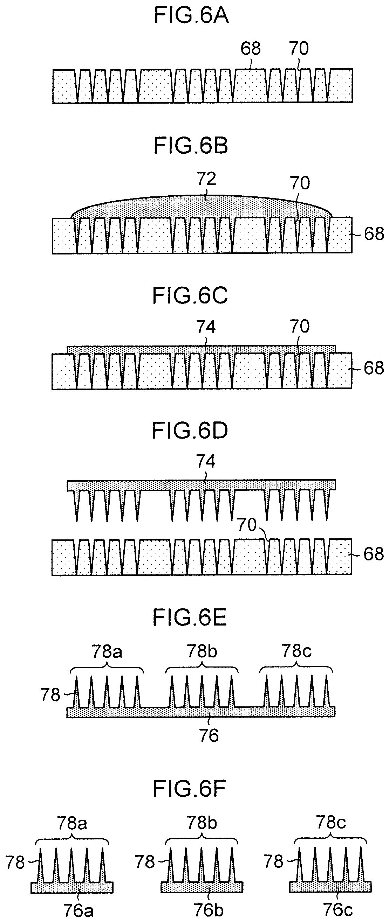

Subsequently, a method of manufacturing a pattern sheet by using the mold 68 manufactured by the method of manufacturing described above will be described. Figs. 6A to 6F are a process chart of manufacturing the pattern sheet 76.

### [Step of supplying Polymer Solution]

Fig. 6A illustrates a state where the mold 68 is prepared. The mold 68 is manufactured by the method of manufacturing a mold, described above.

Fig. 6B illustrates a step of supplying a polymer solution 72 to the recessed pattern 70 of the mold 68.

It is preferable to use a water-soluble material as a material of the polymer solution 72 forming the pattern sheet 76. In addition, it is preferable to use a biocompatible resin as a resin polymer of the polymer solution to be used for manufacturing of a pattern sheet. As this kind of resin, it is preferable to use a saccharide such as glucose, maltose, pullulan, sodium chondroitin sulfate, hyaluronate sodium, and hydroxyethyl starch, a protein such as gelatin, or a biodegradable polymer such as polylactide, and lactic acid-glycolic acid copolymer. Among them, a gelatin-based material can be suitably used because it has adhesion to many base materials and its robust gel strength as a gelling material, so that it can be brought into close contact with a base material, and the pattern sheet 76 can be released by using the base material (not illustrated) when the pattern sheet 76 is released from the mold 68. While a concentration of the resin polymer is different depending on materials, it is preferable that the concentration of a resin polymer contained in a polymer solution without a medicine is 10 to 50 mass %. In addition, a solvent used for dissolution is not necessarily warm water as long as the solvent has volatility, and thus methyl ethyl ketone (MEK), alcohol, or the like can be used. In the solution of a polymer resin, a medicine to be supplied into the body can be dissolved together in accordance with usage. It is preferable that polymer concentration (concentration of a polymer except a medicine if the medicine itself is a polymer) in the polymer solution containing a medicine is 0 to 30 mass %.

In a case of using a water-soluble high molecule (e.g. gelatin), a method of preparing a polymer solution may include the following: dissolving water-soluble powder in water and then adding a medicine into the solution; and pouring water-soluble high-molecular powder into a liquid in which a medicine is dissolved. If the water-soluble high molecule is difficult to be dissolved in water, it may be dissolved while being heated. It is preferable to heat the water at a temperature of about less than 60°C while the temperature can be appropriately determined depending on a kind of the high molecular material. A viscosity of the polymer resin solution containing a medicine is preferably 100 Pa·s or less, and more preferably is 10 Pa·s or less. A viscosity of the solution without a medicine is preferably 2000 Pa·s or less, and more preferably is 1000 Pa·s or less. The appropriate adjustment of the viscosity of the polymer resin solution allows the solution to be easily injected into needle-like recessed portions of a mold.
For example, the viscosity of the polymer resin solution can be measured with a tube type viscometer, a falling ball viscometer, a rotational viscometer, or a vibration type viscometer.

The medicine to be contained in the polymer solution is not limited if it has a function of a medicine. It is preferable that the medicine is particularly selected from peptide, protein, nucleic acid, polysaccharide, vaccine, a medicinal compound belonging to a water-soluble low-molecular compound, or a cosmetic ingredient.

A method of injecting this kind of resin polymer solution into the mold 68 includes an application using a spin coater, for example.

Since a tip of each of recessed portions of the recessed pattern 70 of the mold 68 has a through-hole penetrating the mold 68, air in each of the recessed portions of the recessed pattern 70 can be released through the through-hole. Thus, the polymer solution 72 can be easily poured into the recessed portions of the mold 68. In addition, it is preferable to perform this step under a reduced pressure state.

### [Step of drying]

Fig. 6C illustrates a step of drying the polymer solution 72 to form a polymer sheet 74. For example, the polymer solution 72 can be dried by blowing air to the polymer solution 72 supplied to the mold 68. The polymer sheet 74 means the polymer solution 72 in a state after a desired drying process has been applied thereto. The amount of moisture of the polymer sheet 74 and the like are appropriately set. If the amount of moisture of the polymer decreases too much by drying, the polymer sheet 74 tends to be difficult to be released, thus it is preferable to keep the amount of moisture at which an elasticity is maintained.

### [Step of releasing Polymer Sheet]

Figs. 6D and 6E illustrate a state where the polymer sheet 74 is released from the mold 68 to form the pattern sheet 76, and Fig. 6F illustrates a step of cutting the pattern sheet 76 to form individual pattern sheets 76a, 76b, and 76c.

The pattern sheet 76 released from the mold 68 is set in a cutting device (not illustrated), and cutting positions of the pattern sheet 76 are determined. The cutting positions are basically determined so that areas 78a, 78b, and 78c, each having the protrusion pattern 78, are acquired. As illustrated in Fig. 6F, the plurality of individual pattern sheets 76a, 76b, and 76c are formed by cutting the pattern sheet 76.

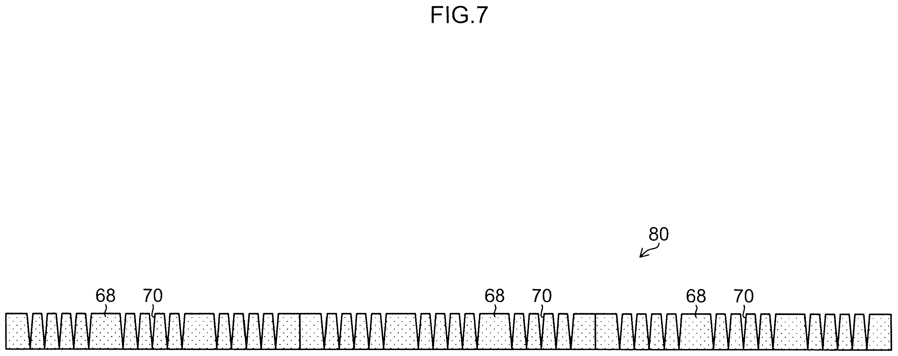

While the method of manufacturing the pattern sheet 76 by using the mold 68 is illustrated in Figs. 6A to 6F, as shown in Fig. 7, the pattern sheet also can be manufactured by manufacturing the assembled mold 80, in accordance with the current invention, in which the plurality of molds 68 are joined to have a face with an increased area in which the recessed pattern 70 is formed. The assembled mold 80 can be manufactured by a method similar to the method of manufacturing the first assembled mold, described above.

Manufacturing the pattern sheet by using the assembled mold 80 enables a pattern sheet with a large area to be manufactured by one manufacturing, and thus the productivity can be improved.

In the present embodiment, there is described a case where the polymer sheet 74 is formed by filling the polymer solution 72 into the recessed pattern 70 of the mold 68 and drying the filled polymer solution 72, however, the present invention is not limited to the case.

For example, the recessed pattern 70 of the mold 68 is filled with a polymer solution containing a medicine and the polymer solution is dried, and then the recessed pattern 70 of the mold 68 is filled with a polymer solution containing no medicine and the polymer solution is dried to enable the polymer sheet 74 to be formed.

The mold 68 is sometimes preferable to be used only once and then disposed. In a case where the pattern sheet 76 is used for medical supplies, it is preferable that the pattern sheet is disposable in consideration of safety of the manufactured pattern sheet 76, with respect to a living body. By allowing the mold 68 to be disposable, there is no longer need for cleaning of the mold 68, and thus costs caused by cleaning can be reduced. Particularly, in a case the pattern sheet 76 is used for medical supplies, high detergency is required, which increases cleaning costs.

A shape of a protrusion of the manufactured pattern sheet 76 is not particularly limited as long as a tip tapers. For example, the shape can be a conic shape, or a pyramid shape such as a triangular pyramid and a quadrangular pyramid. In addition, the shape can be formed by a needle section with a tapered shape and a frustum section connected to the needle section.

A height of the protrusion of the protrusion pattern is within a range of not less than 100 µm and not more than 2000 µm, and is preferably within a range of not less than 200 µm and not more than 1500 µm.

Since the manufactured pattern sheet 76 having the protrusion pattern 78 is a duplicate of the model 40 having the protrusion pattern 42, forming a shape of the protrusion pattern 42 of the model 40, or a shape of the protrusion pattern 12 of the master plate 10 in a desired shape enables the manufactured protrusion pattern 78 of the pattern sheet 76 to be formed in the desired shape.

## Claims

1. A method of manufacturing a mold (68) having a recessed pattern (70), the method comprising:
applying a thermosetting resin solution (58) on a surface of a model (40) having a protrusion pattern (42) to form a thermosetting resin film (60);
bringing a separation sheet (66) into close contact with one face of a base plate (64) to prepare the base plate (64);
providing a gap adjustment mechanism (52) around the model (40) having the protrusion pattern (42) and pressing the separation sheet side of the base plate (64) on the gap adjustment mechanism (52) and the thermosetting resin film (60);
heating and curing the thermosetting resin film (60) to form a mold (68) in a state where the base plate (64) is in contact with the thermosetting resin film (60);
releasing the base plate (64); and
releasing the mold (68) from the model (40) in a state where the separation sheet (66) is attached to the mold (68) and then releasing the separation sheet (66) from the mold (68),
wherein the gap adjustment mechanism (52) has a height at which a tip of the protrusion pattern (42) sticks into the separation sheet (66) when the base plate (64) is pressed on the gap adjustment mechanism (52), and
a plurality of molds (68) are joined to form an assembled mold (80).

2. The method of manufacturing a mold (68) according to claim 1, wherein the separation sheet (66) is a fluorocarbon rubber sheet, or a silicone resin sheet to which a release treatment has been applied.

3. The method of manufacturing a mold (68) according to claim 1 or 2, wherein the base plate (64) is provided with an air suctioning mechanism and an air injecting mechanism to bring the separation sheet (66) into close contact with the base plate (64) or release the separation sheet (66) from the base plate (64).

4. The method of manufacturing a mold (68) according to any one of claims 1 to 3, wherein the model (40) having the protrusion pattern (42) is a metal electroformed model (40) formed by electro forming from a first mold (30) having a recessed pattern (22) which has a reversed shape of the protrusion pattern (42).

5. The method of manufacturing a mold (68) according to any one of claims 1 to 4, wherein
the model (40) having a protrusion pattern (42) includes a plurality of areas each provided with a protrusion pattern, and
the thermosetting resin solution (58) is applied by a slit nozzle in a width of the protrusion pattern.

6. A method of manufacturing a pattern sheet (76) having a protrusion pattern (78), the method comprising:
supplying a polymer solution (72) to a recessed pattern (70) of a mold (68) manufactured by the method of manufacturing a mold (68) according to any one of claims 1 to 5;
drying the polymer solution (72) to form a polymer sheet (74); and
releasing the polymer sheet (74) from the mold (68).

7. The method of manufacturing a pattern sheet (76) according to claim 6, wherein the polymer solution (72) is a water-soluble material.

8. The method of manufacturing a pattern sheet (76) according to claim 6 or 7, wherein
in supplying the polymer solution (72), the plurality of the molds (68) are joined to form an assembled mold (80) with an increased area of a face in which the recessed pattern is formed, and then the polymer solution (72) is supplied to the assembled mold (80).

## Patentansprüche

1. Verfahren zum Fertigen einer Form (68) mit einem vertieften Muster (70), umfassend:
Aufbringen einer Duroplastlösung (58) auf eine Oberfläche eines Modells (40) mit einem Vorsprungsmuster (42), um eine Duroplastschicht (60) zu bilden;
In-engen-Kontakt-bringen eines Trennflachstücks (66) mit einer Seite einer Basisplatte (64), um die Basisplatte (64) vorzubereiten;
Bereitstellen eines Lückeneinstellmechanismus (52) um das Modell (40) mit dem Vorsprungsmuster (42) und Andrücken der Trennflachstück-Seite der Basisplatte (64) an den Lückeneinstellmechanismus (52) und die Duroplastschicht (60);
Erhitzen und Aushärten der Duroplastschicht (60) zum Bilden einer Form (68) in einem Zustand, in welchem die Basisplatte (64) in Berührung mit der Duroplastschicht (60) steht;
Ablösen der Basisplatte (64); und
Lösen der Form (68) von dem Modell (40) in einem Zustand, in welchem das Trennflachstück (66) an der Form (68) anhaftet, und anschließendes Lösen des Trennflachstücks (66) von der Form (68),
wobei der Lückeneinstellmechanismus (52) eine Höhe aufweist, bei der eine Spitze des Vorsprungsmusters (52) in dem Trennflachstück (66) steckt, wenn die Basisplatte (64) an den Lückeneinstellmechanismus (52) gedrückt wird, und
mehrere Formen (68) zur Bildung einer zusammengesetzten Form (80) vereint werden.

2. Verfahren nach Anspruch 1, bei dem das Trennflachstück (66) ein Fluorkohlenstoffgummi-Flachstück oder ein Silikonharz-Flachstück ist, an dem eine Ablösebehandlung vorgenommen wurde.

3. Verfahren nach Anspruch 1 oder 2, bei dem die Basisplatte (64) mit einem Luftansaugmechanismus und einem Lufteinblasmechanismus ausgestattet ist, um das Trennflachstück (66) in enge Berührung mit der Basisplatte (64) zu bringen oder das Trennflachstück (66) von der Basisplatte (64) zu lösen.

4. Verfahren nach einem der Ansprüche 1 bis 3, bei dem das Modell (40) mit dem Vorsprungsmuster (42) ein durch Elektrogalvanisieren geformtes Metallmodell (40) ist, hergestellt durch Galvanisierformen aus einer ersten Form (30) mit einem vertieften Muster (22), welches eine Umkehrform des Vorsprungsmusters (42) besitzt.

5. Verfahren nach einem der Ansprüche 1 bis 4, bei dem
das Modell (40) mit einem Vorsprungsmuster (42) mehrere, jeweils mit einem Vorsprungsmuster versehene Bereiche enthält, und
die Duroplastlösung (58) mittels einer Schlitzdüse einer dem Vorsprungsmuster entsprechenden Breite aufgebracht wird.

6. Verfahren zum Fertigen eines Musterflachstücks (76) mit einem Vorsprungsmuster (78), umfassend:
Aufbringen einer Polymerlösung (72) auf ein vertieftes Muster (70) einer Form (68), die hergestellt wurde nach dem Verfahren zum Fertigen einer Form (68) nach einem der Ansprüche 1 bis 5;
Trocknen der Polymerlösung (72) zum Bilden eines Polymerflachstücks (74); und
Ablösen des Polymerflachstücks (74) von der Form (68).

7. Verfahren nach Anspruch 6, bei dem die Polymerlösung (72) ein wasserlösliches Material ist.

8. Verfahren nach Anspruch 6 oder 7, bei dem beim Zuführen der Polymerlösung (72) mehrere Formen (68) vereint werden, um eine zusammengesetzte Form (80) mit einer erhöhten Flächengröße einer Seite zu bilden, in welcher das vertiefte Muster gebildet wird, und anschließend die Polymerlösung (72) auf die zusammengesetzte Form (80) aufgebracht wird.

## Revendications

1. Procédé de fabrication d'un moule (68) présentant un motif en retrait (70), le procédé comprenant les étapes suivantes :
appliquer une solution de résine thermodurcissable (58) sur une surface d'un gabarit (40) présentant un motif de protubérance (42) pour former un film de résine thermodurcissable (60) ;
amener une feuille de séparation (66) en contact étroit avec une face d'une plaque de base (64) pour préparer la plaque de base (64) ;
fournir un mécanisme de réglage d'espace (52) autour du gabarit (40) présentant le motif de protubérance (42), et presser le côté de feuille de séparation de la plaque de base (64) sur le mécanisme de réglage d'espace (52) et le film de résine thermodurcissable (60) ;
chauffer et durcir le film de résine thermodurcissable (60) pour former un moule (68) dans un état où la plaque de base (64) est en contact avec le film de résine thermodurcissable (60) ;
démouler la plaque de base (64), et
démouler le moule (68) du gabarit (40) dans un état où la feuille de séparation (66) est attachée au moule (68), puis démouler la feuille de séparation (66) du moule (68),
dans lequel le mécanisme de réglage d'espace (52) présente une hauteur à laquelle une pointe du motif de protubérance (42) vient se mettre dans la feuille de séparation (66) lorsque la plaque de base (64) est pressée sur le mécanisme de réglage d'espace (52), et
une pluralité de moules (68) sont joints pour former un moule assemblé (80).

2. Procédé de fabrication d'un moule (68) selon la revendication 1, dans lequel la feuille de séparation (66) est une feuille de caoutchouc fluoré, ou une feuille de résine de silicone sur laquelle un traitement de démoulage a été appliqué.

3. Procédé de fabrication d'un moule (68) selon la revendication 1 ou 2, dans lequel la plaque de base (64) est dotée d'un mécanisme d'aspiration d'air et d'un mécanisme d'injection d'air pour amener la feuille de séparation (66) en contact étroit avec la plaque de base (64), ou pour démouler la feuille de séparation (66) de la plaque de base (64).

4. Procédé de fabrication d'un moule (68) selon l'une quelconque des revendications 1 à 3, dans lequel le gabarit (40) présentant le motif de protubérance (42) est un gabarit électroformé métallique (40) formé par électroformage à partir d'un premier moule (30) présentant un motif en retrait (22), lequel présente une forme inversée du motif de protubérance (42).

5. Procédé de fabrication d'un moule (68) selon l'une quelconque des revendications 1 à 4, dans lequel
le gabarit (40) présentant un motif de protubérance (42) inclut une pluralité de zones, chacune dotée d'un motif de protubérance, et
la solution de résine thermodurcissable (58) est appliquée par une buse à fente dans une largeur du motif de protubérance.

6. Procédé de fabrication d'une feuille à motif (76) présentant un motif de protubérance (78), le procédé comprenant les étapes suivantes :
alimenter une solution polymère (72) vers un motif en retrait (70) d'un moule (68) fabriqué par le procédé de fabrication d'un moule (68) selon l'une quelconque des revendications 1 à 5 ;
sécher la solution polymère (72) pour former une feuille polymère (74), et
démouler la feuille polymère (74) du moule (68).

7. Procédé de fabrication d'une feuille à motif (76) selon la revendication 6, dans lequel la solution polymère (72) est un matériau soluble dans l'eau.

8. Procédé de fabrication d'une feuille à motif (76) selon la revendication 6 ou 7, dans lequel
lors de l'alimentation de la solution polymère (72), la pluralité des moules (68) sont joints pour former un moule assemblé (80) présentant une zone augmentée d'une face dans laquelle le motif en retrait (70) est formé, puis la solution polymère (72) est alimentée vers le moule assemblé (80).
